# EUROPEAN PATENT APPLICATION

(11) **EP 4 523 544 A1**
(43) Date of publication of application: **19.03.2025**
(21) Application number: 23197419.7
(22) Date of filing: 14.09.2023
(51) Int. Cl.: A23J 1/00, A23J 3/20, A23J 3/22, C12N 1/14

(54) **MYCELIUM FERMENTATION FOR GENERATION OF EDIBLE PROTEINS**

(71) Applicant: Esencia Foods UG (haftungsbeschränkt), 10249 Berlin (DE)
(72) Inventor: KAYE, Hendrik Thilo, 13353 Berlin (DE); SCOCOZZA, Bruno Nicolás, 08006 Barcelona (ES)
(74) Representative: Stellbrink & Partner Patentanwälte mbB

(57) **Abstract**

The present invention relates to a mycelium-containing edible composite product, the product comprising a fungal mycelium-containing biomass grown on a fermentable substrate, wherein the mycelium-containing biomass comprises a non-toxic edible fungal mycelium. Furthermore, the present invention also relates to method for producing the mycelium-containing product, the method comprising: inoculating a fermentable substrate with at least one fungal strain, and fermenting the inoculated the fermentable substrate to generate a composite product comprising a mycelium-containing biomass, wherein the fermenting step is a solid-state fermentation process. Moreover, the invention relates to a system for producing a mycelium-containing product, wherein the system is configured to ferment a fermentable substrate to generate a composite product comprising a mycelium-containing biomass, wherein the fermentable substrate is inoculated with at least one fungal strain.

## Description

### Field

The invention lies in the field of fungal mycelium-containing edible proteins. The goal of the present invention is to provide a fungal mycelium based edible proteins. More particularly, the present invention relates to a system for producing fungal mycelium-containing proteins, a method performed in such a system and corresponding use of the system.

### Introduction

On the one hand, meat, fish and seafood consumption is globally increasing, mainly driven by a growing population and increasing average household incomes. On the other hand, non-animal-based diets are an accelerating trend around the globe. Main motivations to switch from meat, fish and seafood-based diets to non-animal-based diets comprise environmental awareness, health concerns, and animal welfare concerns, but also food security concerns as the population keeps on growing. At the same time, consumers adhere to traditional cooking recipes and cultural customs.

Against this background, it is apparent that replacing fish, seafood, and meat products with analogues (also called alternative proteins) is key to support the transition to a more sustainable food system. Sources for these alternative proteins comprise plants, algae, and fungi.

The most common approaches use peas, wheat or soy as a source protein for meat or fish and seafood analogues. Obtaining the source protein as well as creating a meat or fish and seafood analogue out of it requires extreme processing, resulting in high production costs. Further, taste, texture, nutritional value and sustainability are compromised.

Fungi-based analogues are a recent, emerging category of analogues. The mycelium, the vegetative part of the fungus, grows naturally from the bottom up and/or around a so-called feedstock (or substrate), which results in a desirable texture and mouthfeel. Further, due to the naturally occurring flavor components of the fungus, fungi-based products do not require strong masking of unpleasant off-notes known from soy, pea and wheat-based analogues. Current approaches to produce fungi-based meat, fish and seafood alternatives often rely on growing mycelium submerged in a large steel bioreactor. After harvesting the mycelium biomass, various post-processing steps follow such as cutting, heating, mixing with additives, extruding, and shaping into food products. This process has been introduced to the market by Quorn and is nowadays also performed in this or similar ways among others by Boulder or Prime Roots. Due to the process as such, this approach does not allow to make best use of the of the naturally grown structure.

CN 114 929 256 A refers to method for producing synthetic meat by recombinant expression of muscle protein in an edible biological host, disclosing in particular a method for manufacturing synthetic meat, which can overcome the problem that a large amount of precious water, food and large agricultural area are consumed in the traditional meat production practice.

US 2023 0084 699 A1 refers to a method of forming an edible meat substitute product includes growing fungal cells in a growth media such that the fungal cells produce a mycelium mass having a protein content of greater than 40 wt % of a dry mass of the mycelium mass, wherein the method includes separating the mycelium mass from the growth media, and disposing the mycelium mass on a base of a mold.

CA 3 155 385 A1 refers to a mycelium in the form of an edible aerial mycelium that is suitable for use as a food product, including a food ingredient for making mycelium-based food, such as bacon, and a method of making an edible aerial mycelium suitable for use as a food product, including a food ingredient.

WO 2020 074 782 A1 refers to a food product comprising non-toxic edible fungal mycelium as a binding agent for food particles, using of fungal mycelium as a binding agent for food particles, and a method for the production of a food product comprising non-toxic edible fungal mycelium.

WO 2022 157 326 A1 refers to a food product comprising fungi biomass and at least one food additive, the fungi biomass comprising a filamentous mycelium network wherein the at least one food additive is present in an amount of 0.05% by weight or more of the total weight of the fungi biomass and food additive, wherein the at least food additive is integrated in the filamentous mycelium network.

Overall, current approaches and efforts on manufacturing processes for producing alternatives to animal-based proteins still do neither result in textures nor flavors that would yield a convincing alternative.

### Summary

In light of the above, it is therefore an object of the present invention to overcome or at least to alleviated the shortcomings and disadvantages of the prior art. More particularly, it is an object of the present invention to provide a method and a corresponding system for generating a mycelium-containing edible proteins with improved texture and flavor.

These objects are met by the present invention.

In a first aspect, the invention relates to a mycelium-containing edible composite product, the product comprising a fungal mycelium-containing biomass grown on a fermentable substrate, wherein the mycelium-containing biomass may comprise a non-toxic edible fungal mycelium.

A fermentable substrate inoculated with a at least fungal strain is particularly advantageous, as it allows a mycelium growing on the fermentable substrate by feeding itself from the fermentable substrate.

In one embodiment, the at least one fungal strain may comprise at least one filamentous fungus from Ascomycota and/or Zygomycota phylum, preferably from a genus selected from a group comprising Aspergillus, Rhizopus, Fusarium, Neurospora and Penicillium. Moreover, the at least one filamentous fungus may comprise species selected from a group comprising Aspergillus oryzae, Aspergillus sojae, Rhizopus oryzae, Rhizopus oligosporus, Fusarium venetatum, Penicillium camemberti, Neurospora Intermedia, and Neurospora crassa. Additionally or alternatively, the at least one filamentous fungus may comprise a fungus selected from at least one of groups of: Acidophiles such as Aureobasidium pullans, Thermophiles such as Phycomycetaces blakesleeanus, and Halophiles such as Talaromyces leycettanus.

In a further embodiment, the fungal mycelium-containing biomass may comprise at least one fungal strain from Basidiomycetes division, preferably from a genus selected from a group comprising: Agaricus, Trametes, Fomes, Pleurotus, Pholiota, Lentinula, Ganoderma, Hericium, Morchella. Moreover, the at least one fungal strain may comprise a species of: Agaricus bisporus, Trametes versicolor, Fomes fomentarius, Ganoderma sessile, Pleurotus pulmonarius, Pleurotus ostreatus, Pholiota nameko, Lentinula edodes, Ganoderma lucidum, Ganoderma sinense, Hericium coralloides, Hericium erinaceus, Pleurotus eryngii, and Morchella esculenta.

In one embodiment, the fermentable substrate may comprise at least one nutrient suitable for growing fungal mycelium.

In a further embodiment, the fermentable substrate may comprise a substrate type comprising at least one of: secondary output of food industry, organic substrates from legume family, pulses from legume family, and pulse derivatives. Additionally or alternative, the pulses from legume family may comprise at least one of: soy (Glycine Max), white beans (Phaseolus vulgaris), chickpeas (Cicer arietinum), red beans (Phaseolus vulgaris), broad beans (Vicia faba, family Fabaceae), fava beans (Vicia faba), quinoa (Chenopodium quinoa), lupines (Lupinus), and peas (Pisum sativum). Moreover, the secondary output of food industry may comprise at least one of: okara, beetroot pomace, apple pomace, tomato pomace, textured vegetable proteins, wheat bran, coconut press cake, rapeseed oil press cake, linseed oil press cake, and chickpea bran.

In one embodiment, the pulses derivatives may comprise at least one of: pulse-derived-flours, pulse-derived granulates, and texturized vegetable protein (TVP).

Moreover, the fermentable substrate may comprise a substrate geometry comprising a particle geometry comprising particle sizes between 0.005 mm and 50 mm, preferably between 0.05 mm and 30 mm, more preferably between 0,05 mm and 10 mm. The fermentable substrate may comprise at least one particle comprising the particle sizes, preferably at least two particles comprising the particle sizes. The fermentable substrate may comprise a mix of the at least two particles. The at least two particles may comprise different sizes. In one embodiment, the fermentable substrate geometry may be different from an original shape of the fermentable substrate type. For instance, the fermentable substrate geometry may comprise a shape different than the original legume shape, including but not limited to an arrangement of particles of flake-like three-dimensional geometry, as well as rod-like / thread-like particles, that may be elongated in one dimension and thin along the other two dimensions.

Furthermore, particle geometry of the fermentable substrate geometry may comprise at least one geometrical shape of: octahedral, cylindrical, spherical, pyramidal, tetrahedral, conical, rectangular prism, icosahedral, dodecahedral, cuboctahedral, helical, ellipsoid, parabolic, hyperbolic, rhombic dodecahedron, cone frustum, cuboctahedron, toroidal, triangular prism, elliptical cylinder, pentagonal prism, crescent, oblate spheroid.

In a further embodiment, wherein the product may comprise a plurality of air pockets comprising an air pocket diameter between 0.05 mm and 5 mm.

In one embodiment, the fermentable substrate may comprise a culture supplement comprising at least one nutrient comprising at least one of: peptones sodium aspartate, malt extract, nutritional yeast, and ammonium nitrate.

In another embodiment, the product may comprise at least one flavoring component comprising at least one of: vegan compound, artificial compound, algae derived compound, and natural industrially available compound . For instance, the algae derive compound may be in a form of a algae broth.

Further, the fungal mycelium-containing biomass may be at least partially hydrophobic.

In one embodiment, the product may comprise a longitudinal direction, a transversal direction and a height, wherein the longitudinal direction may be larger than the transversal direction, the longitudinal direction comprising a longitudinal product dimension of at least 5 mm, preferably between 10 mm and 140 mm, and the height of at least 1 mm, preferably between 2 mm and 50 mm.

In another embodiment, the product may comprise a surface irregularity of at least 0.1 mm, preferably between 1 mm and 20 mm.

In a further embodiment, the product may comprise a product shape comprising one of: square shape, a meat fillet shape, a meat stripe shape, a fish piece shape, a fish stripe shape, a fish burger shape, a shrimp shape, a prawn shape, a Saint Jacques / Scallop shape, a fish shape, and fish fillet shape.

In one embodiment, the product may comprise a product texture resembling at least one of: seafood texture, fish texture, squid texture, eel texture, prawn texture, shrimp texture, Saint Jacques scallop texture, Cod fish texture, Salmon texture, Atlantic pollock texture, Bream texture, chicken breast texture, chicken leg texture, Beef fillet texture, pork fillet texture, seafood, fish, eel, prawn, shrimp, Cod fillet, Salmon fillet, Bream fillet, chicken breast, chicken leg, pork fillet, square, meat fillet, meat strip, fish piece, fish burger, shrimp, prawn, Saint Jacques, Scallop, fish, grass carp fillet, Peruvian anchoveta fillet, Silver carp fillet, Common carp fillet, Alaska pollock fillet, Nile tilapia fillet, Bighead carp fillet, Tuna fillet, Seabass fillet.

In another embodiment, the product may comprise at least one functional ingredient with a concentration of at least 0.005% w/w, preferably between 0.01% w/w and 3% w/w. Moreover, the at least one functional ingredient may comprise at least one of: starch such as modified corn starch, fiber such as bamboo fibers, and gum such as acacia gum and konjac gum, and hydrocolloids such as alginate. In another embodiment, the at least one functional ingredient may comprise at least one of: Bacterial polysaccharides such as Gellan, Xanthan; Carrageenan such as Iota-carrageenan, Kappa-carrageenan; Galactomannan such as Locust bean gum, Guar gum; Fibers such as Psyllium, Bamboo, Oat; and Starches such as Modified Starches. Further, the at least one functional ingredient may be incorporated into the fermentable substrate prior to a fermentation step.

Moreover, the product may comprise at least one fatty oil in ranges between 0.1 % w/w and 3 % w/w. The at least one fatty oil comprises at least one of: deodorized coconut oil, linseed oil, rapeseed oil, pumpkin seed oil, microalgae derived oil such as oil from microalgae Schizochytrium sp., and macroalgae derived oil.

In one embodiment, the product may comprise at least one of: eicosapentaenoic acid (EPA), and docosahexaenoic acid (DHA) in a concentration of at least 1 microgram/g, preferably above 5 micrograms/g.

In a further embodiment, the product may comprise at least one supplementary compound comprising at least one of: vitamin, and mineral. The at least one vitamin may comprise at least one of: vitamin B such as B-1, B-2, B-3, B-5, B-6, B-7, B-9, and B-12; vitamin C; vitamin A; vitamin D; vitamin E; and vitamin K. The at least one mineral may comprise at least one of: sodium selenite, potassium iodide, calcium, potassium, sodium, chloride, phosphorus, magnesium, iron, copper, fluoride, selenium, zinc, chromium, molybdenum, iodine, and manganese.

In one embodiment, the at least one functional ingredient may be incorporated into the fermentable substrate after the fermentation step.

Addition of the at least one functional ingredient is particularly advantageous, as it allows to create a specific texture and/or mouthfeel. E.g., addition of iota carrageenan can provide a more slithery, melting mouthfeel, as occurring in several fish, while bamboo fibers, rice flakes, can create a more fibrous feeling.

In one embodiment, wherein the at least one ingredient may be added before the fermentation step. In another embodiment, the at least one ingredient may be added after the fermentation step. In a further embodiment, the at least one ingredient may be added after: the fermentation step and a heat treatment may be performed, which is notably advantageous as it allows to create a gel-like texture and improve water retention capacity of the mycelium-comprising product.

Addition of the at least one functional ingredient is notably advantageous, as it allows to avoid weight loss in the range of 10% to 50% w/w that occurs due to fermentation by water evaporation and thus improves water-retention capacity of the mycelium-comprising product. As a result, dehydration during the fermentation process may be avoided leading to a consistent humidity during the fermentation and therefore consistent mycelia growth as well as the formation of undesired microorganisms may be inhibited.

In one embodiment, the product may comprise at least one food additive may comprise at least one of: preservative, flavor enhancer, colorant, emulsifier, stabilizer, thickener, and sweetener.

In another embodiment, the product may be free from additives, particularly free of texturizing elements such as forms of: microcrystalline cellulose (E460), methyl cellulose (E461), ethyl cellulose (E462), hydroxypropyl cellulose (E463), hydroxylpropyl methyl cellulose (E464), ethyl methyl cellulose (E465), sodium carboxy methyl cellulose (E466), cross-linked carboxy methyl cellulose (E468), enzymatically hydrolysed arboxy methyl cellulose (E469) and hydroxyethyl cellulose (E1525).

In a second aspect, the invention relates to a method for producing a mycelium-containing product, the method comprising: inoculating a fermentable substrate with at least one fungal strain, and fermenting the inoculated the fermentable substrate to generate a composite product comprising a mycelium-containing biomass.

In one embodiment, the mycelium-containing product may comprise a fungal mycelium and a fungal mycelium-containing biomass on the fermentable substrate, wherein the mycelium-containing biomass may comprise a non-toxic edible fungal mycelium. Additionally or alternatively, the method may comprise performing the fermenting step providing to the fermentable substrate the at least one fungal strain comprising at least one filamentous fungus from Ascomycota and/or Zygomycota phylum, preferably from a genus selected from t a group comprising Aspergillus, Rhizopus, Fusarium, Neurospora and Penicillium. Further, the at least one filamentous fungus may comprise species selected from a group comprising Aspergillus oryzae, Rhizopus oryzae, Rhizopus oligosporus, Fusarium venetatum, Penicillium camemberti, Neurospora Intermedia, and Neurospora crassa. Moreover, the at least one filamentous fungus may comprise a fungus selected from at least one of groups of: Halophiles such as Talaromyces leycettanu, Thermophiles such as Phycomycetaces blakesleeanus, and Acidophiles such as Aureobasidium pullans.

In one embodiment, the fungal mycelium-containing biomass may comprise at least one fungal strain from Basidiomycetes division, preferably from a genus selected from a group comprising Agaricus, Trametes, Fomes, Ganoderman, Pleurotus, Pholiota, Lentinula, Ganoderma, Hericium, Morchella. Additionally or alternatively, the at least one fungal strain may comprise a species of: Agaricus bisporus, Trametes versicolor, Fomes fomentarius, Ganoderma sessile, Pleurotus pulmonarius, Pleurotus ostreatus, Pholiota nameko, Lentinula edodes, Ganoderma lucidum, Ganoderma sinense, Hericium coralloides, Hericium erinaceus, Pleurotus eryngii, and Morchella esculenta.

In a further embodiment, the method may comprise growing fungal mycelium, wherein the method further may comprise providing to the fermenting step at least one at least one nutrient suitable for growing the fungal mycelium. Additionally or alternatively, the at least one nutrient may be comprised by the fermentable substrate.

In one embodiment, the fermentable substrate may comprise a substrate type comprising at least one of: Secondary output of food industry, Organic substrates from legume family, Pulses from legume family, and Pulse derivatives. Additionally or alternatively, the pulses may comprise at least one of: soy, white beans, chickpeas, red beans, broad beans, quinoa, and peas. In one embodiment, the secondary output of food industry may comprise at least one of: okara, beetroot pomace, apple pomace, tomato pomace, textured vegetable proteins, wheat bran, coconut press cake, rapeseed oil press cake, linseed oil press cake, and chickpea bran. For instance, the second output may comprise a press cake which provide to the product a giving aroma, aroma note and/or flavor. For example, the press cake may comprise pistachios, which is surprisingly advantageous, as it provides a fish note to the product. It should be understood that a plurality of nuts and seed oil press cakes may be used for this purpose.

In one embodiment, the method may comprise controlling at least one fermentation parameter comprising at least one of: temperature between 25 °C and 35 °C, and relative humidity between 35% and 95%.

In one embodiment, the fermentable substrate may comprise a substrate geometry comprising a particle geometry comprising particle sizes between 0.005 mm and 50 mm, preferably between 0.05 mm and 30 mm, more preferably between 0.05 mm and 10 mm.

Further, the fermentable substrate geometry may be different from an original shape of the fermentable substrate type. Additionally or alternatively, the method may comprise changing the original shape of the fermentable substrate type into the fermentable substrate geometry comprising an arrangement as particles comprising at least one of: flake-like three-dimensional geometry particles; rod-like particles elongated in a first dimension and thin along at least one of: a second dimension and a third dimension; thread-like particles elongated in a first dimension and thin along at least one of: a second dimension and a third dimension.

In one embodiment, the method may comprise providing the product with a plurality of air pockets comprising an air pocket diameter between 0.05 mm and 5 mm. Moreover, the method may comprise controlling the fermentable substrate geometry.

In one embodiment, the method may comprise adding at least one functional ingredient with a concentration of at least 0.005% w/w, preferably between 0.01% w/w and 3% w/w. Additionally or alternatively, the at least one functional ingredient may comprise at least one of: starch such as modified corn starch, fiber such as bam, and gum such as acacia gum and konjac gum, and hydrocolloids such as alginate. Moreover, the at least one functional ingredient may comprise at least one of: Bacterial polysaccharides such as Gellan, Xanthan; Carrageenan such as Iota-carrageenan, Kappa-carrageenan; Galactomannan such as Locust bean gum, Guar gum; and Fibers such as Psyllium, Bamboo, Oat. Additionally or alternatively, the method may comprise incorporating the at least one functional ingredient into the fermentable substrate prior to the fermenting step.

Moreover, the method may comprise incorporating the at least one functional ingredient into the fermentable substrate after the fermenting step. Additionally or alternatively, the method may comprise adding the at least one ingredient before the fermenting step. Further, the method may comprise adding the at least one ingredient after the fermenting step and after a treatment step.

Furthermore, the method may comprise adding at least fatty oil in ranges between 0.1% w/w and 3% w/w. The at least one fatty oil may comprise at least one of: deodorized coconut oil, linseed oil, rapeseed oil, pumpkin seed oil, microalgae derived oil such as oil from microalgae Schizochytrium sp., and macroalgae derived oil. For example, the at least one fatty oil may be added as part of a marinade.

In one embodiment, the method may comprise producing fish flavors by burning or heating up the at least one fatty oil. Additionally or alternatively, the heating step comprises heating between 50 °C and 220 °C for at least 1 minutes, preferably between 5 and 15 min.

In another embodiment, the method may comprise adding at least one of: eicosapentaenoic acid (EPA), and docosahexaenoic acid (DHA) in a concentration of at least 1 microgram/g, preferably above 5 micrograms/g.

In a further embodiment, the method may comprise adding at least one supplementary compound comprising at least one of: vitamin, and mineral. The at least one vitamin may comprise at least one of: vitamin B such as B-1, B-2, B-3, B-5, B-6, B-7, B-9, and B-12; vitamin C, vitamin A, vitamin D, vitamin E, and vitamin K. The at least one mineral may comprise at least one of: sodium selenite, potassium iodide, calcium, potassium, sodium, chloride, phosphorus, magnesium, iron, copper, fluoride, selenium, zinc, chromium, molybdenum, iodine, and manganese.

In one embodiment, the method may comprise producing the fermentable substrate for the fermenting step, the method comprising at least one of: grinding at least one ingredient to obtain a grinded material; milling at least one ingredient to obtain a milled material; reshaping at least one of: the grinded material and the milled material; and blending at least one of: the grinded material and the milled material. Further, the method may comprise controlling the fermentable substrate geometry by reshaping at least one of: the grinded material and the milled material. Moreover, the method may comprise controlling step may comprise the blending step preceding the reshaping step.

In one embodiment, the method may comprise controlling a particle size of the at least one ingredient.

In another embodiment, the method may comprise controlling a product porosity via controlling the plurality of air pockets.

In a further embodiment, the method may comprise for the fermenting step providing to the fermentable substrate a culture supplement comprising at least one nutrient comprising at least one of: peptones sodium aspartate, malt extract, nutritional yeast, and ammonium nitrate

In one embodiment, the method may comprise adjusting a product flavor by adding at least one flavoring component comprising at least one of: vegan compound, artificial compound, algae derived compound, and natural industrially available compound.

Furthermore, the fungal mycelium growing may comprise a controlled growing.

In one embodiment, the fermenting step may be a solid-state fermentation process.

In another embodiment, the fungal mycelium-containing biomass may be at least partially hydrophobic.

Moreover, the fermenting step may comprise a fermentation temperature above 25 °C, preferably 25 °C and 35 °C, more preferably between 27 °C and 31 °C.

In one embodiment, the method may comprise controlling a product shape comprising one of: a square shape, a fish shape, seafood, fish, eel, prawn, shrimp, Cod fillet, Salmon fillet, Bream fillet, chicken breast, chicken leg, pork fillet, square, meat fillet, meat strip, fish piece, fish burger, shrimp, prawn, Saint Jacques, Scallop, fish, grass carp fillet, Peruvian anchoveta fillet, Silver carp fillet, Common carp fillet, Alaska pollock fillet, Nile tilapia fillet, Bighead carp fillet, Tuna fillet, and seabass fillet.

In another embodiment, the method may comprise providing the mycelium-containing product with a product texture resembling at least one of: seafood texture, fish texture, squid texture, eel texture, prawn texture, shrimp texture, Saint Jacques scallop texture, Cod fish texture, Salmon texture, Atlantic pollock texture, Bream texture, chicken breast texture, chicken leg texture, Beef fillet texture, pork fillet texture, seafood, fish, eel, prawn, shrimp, Cod fillet, Salmon fillet, Bream fillet, chicken breast, chicken leg, pork fillet, square, meat fillet, meat strip, fish piece, fish burger, shrimp, prawn, Saint Jacques, Scallop, fish, Grass carp fillet, Peruvian anchoveta fillet, Silver carp fillet, common carp fillet, Alaska pollock fillet, Nile tilapia fillet, Bighead carp fillet, Tuna fillet, Seabass fillet.

Further, the method may comprise controlling at least one process parameter of the solid-state fermentation process, the at least one process parameter comprising at least one of: water content of the fermentable substrate, airflow, humidity of the fermentable substrate, pH of the fermentable substrate, carbon dioxide content, and fermentation temperature.

In one embodiment, the method may comprise reducing and/or eliminating microbial contamination in the fermentable substrate by means of at least one substrate pre-treatment comprising at least one of: sterilization, pasteurization, and pH adjustment in the fermentable substrate. Additionally or alternatively, the controlled growing may comprise growing the mycelium substantially linear. Moreover, the controlled growing may comprise a rhizomorphic growing.

In one embodiment, the mycelium-containing product may be according to any of the preceding product embodiments.

In another embodiment, the method may comprise casting the product after the fermenting step.

In one embodiment, the method may comprise fermenting the fermentable material on a substrate in a physical cast or mold. Additionally or alternatively, the physical cast or mold may comprise a plurality of holes. Moreover, the method the physical cast or mold comprising a surface texture mimicking a surface texture of an animal product as well as providing a mouthfeel, for instance, resembling that of when consuming an animal product. Molds may be used, e.g., for shaping vegan shrimps, while these have not been introduced into fermentation. For fermentation, the mold needs to be custom-made for a given fermented biomass and strain since fermenting in a mold may impact heat transfer, humidity, aeration and hence growth of the mycelium. Mycelium-containing product may be casted into after fermentation or fermented in a physical cast/mold that results in a surface texture, so that when the product is cut, breaking occurs in the determined regions/flakes, similar to the product that may be aimed to be mimicked, which therefore determines the structure of the pieces formed during consumption and therefore the texture and mouthfeel of the product. Moreover, the product may be fermented in a mold which has been custom-made for the mycelium-containing product and where the fermentation parameters as well as the amount of fungal inoculum and/or other ingredients have been adapted.

In one embodiment, the method may comprise: processing the product by at least one of: grinding, slicing or extruding the composite product into pieces of a custom geometry, and binding the pieces together, wherein the binding may comprise at least one of: an ordered binding manner, or disordered binding manner. Additionally or alternatively, the method may comprise binding the pieces by incorporating a binding liquid mixture comprising at least one binding agent. Additionally or alternatively, the at least one binding agent may comprise at least one of: transglutaminases, hydrocolloids, sodium alginate, carrageenan, modified starches, methylcellulose or vegetable protein powders. Moreover, the method may comprise: processing the composite after the fermenting step by slicing the composite into pieces of a custom geometry, and binding the pieces by allowing the fungal mycelium to regrow in between the pieces in a second fermentation step to produce the product which notably advantageous to dispense with the use of binding additives or at least to avoid or reduce the use of binding additives.

In one embodiment, the method may comprise processing the composite after the fermenting step by at least one of: cutting, grinding, slicing, or extruding to obtain fibrils with an axial dimension of at least 1 mm, preferably 3 mm, most preferably above 5 mm, and a diameter below 8mm, preferably 5 mm, most preferably 1 mm.

In another embodiment, the method may comprise joining together mycelium composite fibrils comprised by the in an aligned manner, wherein the mycelium composite fibrils may comprise a longitudinal direction, wherein the method may comprise aligning the longitudinal direction by: using at least one binder, or forming an anisotropic matrix by regrowing the fungal mycelium in a second fermentation to produce the product. Additionally or alternatively, the method may comprise distributing in between the joined fibrils at least one fat-containing ingredient.

In one embodiment, the method may comprise fermenting the fermentable material in a mold comprising a custom surface texture, including an overall 3D shape. Additionally or alternatively, the mold may be produced by using at least one of: silicon, thermoforming, and 3D printing.

In a further embodiment, method may comprise fermenting the fermentable material in a mold comprising a negative of an animal origin protein product. Additionally or alternatively, the negative of the animal protein origin product may comprise enhanced surface features with respect to surface features of the animal origin protein product.

In one embodiment, fermentation of the inoculated substrate in a mold that creates incisions/indentations in the product of at least 0.1 mm, preferably between 1 mm and 10 mm. This is notably advantageous, as it allows generation of a texture generated which allows increasing the interaction with the taste buds, giving a sensation of texture, which requires less glutamate, as the reaction surface increases. Furthermore, the incisions or the indentation, which may copy for example textures present in a fish, give a perception in the mouth of irregularity that makes the product feel more realistic. Moreover, if the incision is deep enough, it is notably advantageous, as the product may break easier in that point therefore imparting preferential breaking locations upon cutting. Additionally, it allows enhancing the indentation/roughness features with respect to the original, which is particularly beneficial, as it allows maintaining the shape during further post-processing steps, such as inactivation processes such as pasteurization.

In one embodiment, the method may comprise at least one of: agitating or mixing the inoculated substrate, coating the mycelium after a first fermenting step to generate coated particles, and placing the coated particles in a mold. Additionally or alternatively, the method may comprise coating the mycelium with at least one functional ingredient.

In one embodiment, the method may comprise providing at least one food additive. Additionally or alternatively, the at least one food additive may comprise at least one of: preservative, flavor enhancer, colorant, emulsifier, stabilizer, thickener, and sweetener.

Moreover, the method may comprise adding the at least one food additive during the fermenting step.

Furthermore, method may comprise producing the mycelium-containing product free from additives, particularly free of texturizing elements such as forms of: microcrystalline cellulose (E460), methyl cellulose (E461), ethyl cellulose (E462), hydroxypropyl cellulose (E463), hydroxylpropyl methyl cellulose (E464), ethyl methyl cellulose (E465), sodium carboxy methyl cellulose (E466), cross-linked carboxy methyl cellulose (E468), enzymatically hydrolysed arboxy methyl cellulose (E469) and hydroxyethyl cellulose (E1525).

In one embodiment, in the fermenting step, the method may comprise covering the inoculated substrate with at least one removable covering component. Additionally or alternatively, the at least one removable covering component may comprise a membrane comprising a plurality of porous to allow for oxygen, heat and humidity exchange. Further, the membrane may comprise a layer of a polymeric film.

In one embodiment, the method may comprise mixing at least one particle comprising the particle sizes, preferably at least two particles comprising the particle sizes. Additionally or alternatively, the at least two particles may comprise different particle sizes.

In a third aspect, the invention relates to a system for producing a mycelium-containing product, wherein may be configured to ferment a fermentable substrate to generate a composite product comprising a mycelium-containing biomass, wherein the fermentable substrate is inoculated with at least one fungal strain.

In one embodiment, wherein the composite product may be according to the product as recited herein.

Furthermore, the system may comprise a tray bioreactor configured to ferment a substrate inoculated with at least one fungal strain capable of producing a non-toxic edible fungal mycelium.

In another embodiment, the system may comprise a solid-state drum bioreactor configured to provide movement to particles during mixing step.

In a further embodiment, the system may comprise a continuous or semi-continuous solid-state fermentation bioreactor.

Moreover, the system may comprise at least one fermentation tray comprising a 3D shape. Additionally or alternatively, the at least one fermentation tray may comprise at least one hole configured to allow aeration. Moreover, the at least one fermentation tray may be shaped in form of an animal-origin product, wherein the form may comprise at least one of: seafood, fish, squid, eel, prawn, shrimp, Saint Jacques scallop, Cod, Salmon texture, Atlantic pollock, Bream, chicken breast, chicken leg, Beef fillet, pork fillet, Cod fillet, Salmon fillet, Bream fillet, chicken breast, chicken leg, pork fillet, square, meat fillet, meat strip, fish piece, fish burger, shrimp, prawn, Saint Jacques, Scallop, fish, Grass carp fillet, Peruvian anchoveta fillet, Silver carp fillet, common carp fillet, Alaska pollock fillet, Nile tilapia fillet, Bighead carp fillet, Tuna fillet, Seabass fillet.

In one embodiment, wherein the system may comprise at least one removable covering component configured to cover the inoculated substrate. Further, the at least one removable covering component may comprise a membrane comprising a plurality of porous to allow for oxygen, heat and humidity exchange. Moreover, the membrane may comprise a layer of a polymeric film.

In one embodiment, the system may be configured to carry out the method as recited herein.

In another embodiment, the method comprising prompting the system as recited herein to perform the method as recited herein to produce the product as recited herein.

The invention also relates to the use of the system according as recited herein for carrying out the method as recited herein.

The present technology is also described by the following numbered embodiments.

Below, product embodiments will be discussed. These embodiments are abbreviated by the letter "P" followed by a number. When reference is herein made to a product embodiment, those embodiments are meant.

P1. A mycelium-containing edible composite product, the product comprising a fungal mycelium-containing biomass grown on a fermentable substrate, wherein the mycelium-containing biomass comprises a non-toxic edible fungal mycelium.

P2. The product according to the preceding embodiment, wherein the at least one fungal strain comprises at least one filamentous fungus from Ascomycota and/or Zygomycota phylum, preferably from a genus selected from a group comprising Aspergillus, Rhizopus, Fusarium, Neurospora and Penicillium.

P3. The product according to the preceding embodiment, wherein the at least one filamentous fungus comprises species selected from a group comprising Aspergillus oryzae, Aspergillus sojae, Rhizopus oryzae, Rhizopus oligosporus, Fusarium venetatum, Penicillium camemberti, Neurospora Intermedia, and Neurospora crassa.

P4. The product according to any of the two preceding embodiments, wherein the at least one filamentous fungus comprises a fungus selected from at least one of groups of
Acidophiles such as Aureobasidium pullans,
Thermophiles such as Phycomycetaces blakesleeanus, and
Halophiles such as Talaromyces leycettanus.

P5. The product according to any of the preceding embodiments, wherein the fungal mycelium-containing biomass comprises at least one fungal strain from Basidiomycetes division, preferably from a genus selected from a group comprising Agaricus, Trametes, Fomes, Pleurotus, Pholiota, Lentinula, Ganoderma, Hericium, Morchella.

P6. The product according to the preceding embodiment, wherein the at least one fungal strain comprises a species of: Agaricus bisporus, Trametes versicolor, Fomes fomentarius, Ganoderma sessile, Pleurotus pulmonarius, Pleurotus ostreatus, Pholiota nameko, Lentinula edodes, Ganoderma lucidum, Ganoderma sinense, Hericium coralloides, Hericium erinaceus, Pleurotus eryngii, and Morchella esculenta.

P7. The product according to any of the preceding embodiments, wherein the fermentable substrate comprises at least one nutrient suitable for growing fungal mycelium.

P8. The product according to any of the preceding embodiments, wherein the fermentable substrate comprises a substrate type comprising at least one of
Secondary output of food industry,
Organic substrates from legume family,
Pulses from legume family, and
Pulse derivatives

P9. The product according to the preceding embodiment, wherein the pulses from legume family comprise at least one of:
soy (Glycine Max),
white beans (Phaseolus vulgaris),
chickpeas (Cicer arietinum),
red beans (Phaseolus vulgaris),
broad beans (Vicia faba, family Fabaceae),
fava beans (Vicia faba),
quinoa (Chenopodium quinoa),
lupines (Lupinus), and
peas (Pisum sativum).

P10. The product according to embodiment P9, wherein the secondary output of food industry comprises at least one of:
okara,
beetroot pomace,
apple pomace,
tomato pomace,
textured vegetable proteins,
wheat bran,
coconut press cake,
rapeseed oil press cake,
linseed oil press cake, and
chickpea bran.

P11. The product according to any of the preceding embodiments and with features of embodiment P8, wherein the pulses derivatives comprise at least one of:
pulse-derived-flours,
pulse-derived granulates, and
texturized vegetable protein (TVP).

P12. The product according to any of the preceding embodiments, wherein the fermentable substrate comprises a substrate geometry comprising a particle geometry comprising particle sizes between 0.005 mm and 50 mm, preferably between 0.05 mm and 30 mm, more preferably between 0,05 mm and 10 mm.

P13. The product according to the preceding embodiment, wherein the fermentable substrate comprises at least one particle comprising the particle sizes, preferably at least two particles comprising the particle sizes.

P14. The product according to the preceding embodiment, wherein the fermentable substrate comprises a mix of the at least two particles.

P15. The product according to the preceding embodiment, wherein the at least two particles comprise different sizes.

P16. The product according to any of the four preceding embodiments and with the feature of embodiment P8, wherein the fermentable substrate geometry is different from an original shape of the fermentable substrate type.

P17. The product according to any of the preceding embodiments and with the features of any of embodiments P12 to P16, wherein the particle geometry of the fermentable substrate geometry comprises at least one geometrical shape of: octahedral, cylindrical, spherical, pyramidal, tetrahedral, conical, rectangular prism, icosahedral, dodecahedral, cuboctahedral, helical, ellipsoid, parabolic, hyperbolic, rhombic dodecahedron, cone frustum, cuboctahedron, toroidal, triangular prism, elliptical cylinder, pentagonal prism, crescent, oblate spheroid.

P18. The product according to any of the preceding embodiments, wherein the product comprises a plurality of air pockets comprising an air pocket diameter between 0.05 mm and 5 mm.

P19. The product according to any of preceding embodiments, wherein the fermentable substrate comprises a culture supplement comprising at least one nutrient comprising at least one of:
peptones,
sodium aspartate,
malt extract,
nutritional yeast, and
ammonium nitrate.

P20. The product according to any of the preceding embodiments, wherein the product comprises at least one flavoring component comprising at least one of: vegan compound, artificial compound, algae derived compound, and natural industrially available compound.

P21. The product according to any of the preceding embodiments, wherein the fungal mycelium-containing biomass is at least partially hydrophobic.

P22. The product according to any of the preceding embodiments, wherein the product comprises a longitudinal direction, a transversal direction and a height, wherein the longitudinal direction is larger than the transversal direction, the longitudinal direction comprising a longitudinal product dimension of at least 5 mm, preferably between 10 mm and 140 mm, and the height of at least 1 mm, preferably between 2 mm and 50 mm. P23. The product according to any of the preceding embodiments, wherein the product comprises a surface irregularity of at least 0.1 mm, preferably between 1 mm and 20 mm.

P24. The product according to any of the preceding embodiment, wherein the product comprises a product shape comprising one of: square shape, a meat fillet shape, a meat stripe shape, a fish piece shape, a fish stripe shape, a fish burger shape, a shrimp shape, a prawn shape, a Saint Jacques / Scallop shape, a fish shape, and fish fillet shape.

P25. The product according to any of the preceding embodiments, wherein the product comprises a product texture resembling at least one of: seafood texture, fish texture, squid texture, eel texture, prawn texture, shrimp texture, Saint Jacques scallop texture, Cod fish texture, Salmon texture, Atlantic pollock texture, Bream texture, chicken breast texture, chicken leg texture, Beef fillet texture, pork fillet texture, seafood, fish, eel, prawn, shrimp, Cod fillet, Salmon fillet, Bream fillet, chicken breast, chicken leg, pork fillet, square, meat fillet, meat strip, fish piece, fish burger, shrimp, prawn, Saint Jacques, Scallop, fish, grass carp fillet, Peruvian anchoveta fillet, Silver carp fillet, Common carp fillet, Alaska pollock fillet, Nile tilapia fillet, Bighead carp fillet, Tuna fillet, Seabass fillet.

P26. The product according to any of the preceding embodiments, wherein the product comprises at least one functional ingredient with a concentration of at least 0.005% w/w, preferably between 0.01% w/w and 3% w/w.

P27. The product according to the preceding embodiment, wherein the at least one functional ingredient comprises at least one of:
starch such as modified corn starch,
fiber such as bamboo fibers,
gum such as acacia gum and konjac gum, and
hydrocolloids such as alginate.

P28. The product according to any of the two preceding embodiments, wherein the at least one functional ingredient comprises at least one of:
Bacterial polysaccharides such as Gellan, Xanthan;
Carrageenan such as Iota-carrageenan, Kappa-carrageenan;
Galactomannan such as Locust bean gum, Guar gum;
Fibers such as Psyllium, Bamboo, Oat; and
Starches such as Modified Starches.

P29. The product according to any of the two preceding embodiments, wherein the at least one functional ingredient is incorporated into the fermentable substrate prior to a fermentation step.

P30. The product according to any of the preceding embodiments, wherein the product comprises at least one fatty oil in ranges between 0.1 % w/w and 3 % w/w.

P31. The product according to the preceding embodiment, wherein the at least one fatty oil comprises at least one of:
deodorized coconut oil,
linseed oil,
rapeseed oil,
pumpkin seed oil,
microalgae derived oil such as oil from microalgae Schizochytrium sp., and
macroalgae derived oil.

P32. The product according to any of the preceding embodiments, wherein the product comprises at least one of: eicosapentaenoic acid (EPA), and docosahexaenoic acid (DHA) in a concentration of at least 1 microgram/g, preferably above 5 micrograms/g.

P33. The product according to any of the preceding embodiments, wherein the product comprises at least one supplementary compound comprising at least one of: vitamin, and mineral.

P34. The product according to the preceding embodiment, wherein the at least one vitamin comprises at least one of:
vitamin B such as B-1, B-2, B-3, B-5, B-6, B-7, B-9, and B-12;
vitamin C;
vitamin A;
vitamin D;
vitamin E; and
vitamin K.

P35. The product according to any of the two preceding embodiments, wherein the at least one mineral comprises at least one of: sodium selenite, potassium iodide, calcium, potassium, sodium, chloride, phosphorus, magnesium, iron, copper, fluoride, selenium, zinc, chromium, molybdenum, iodine, and manganese.

P36. The product according to any of embodiments P21 or P22 and with features of the previous embodiment, wherein the at least one functional ingredient is incorporated into the fermentable substrate after the fermentation step.

P37. The product according to the preceding embodiment, wherein the at least one ingredient is added before the fermentation step.

P38. The product according to any of the two preceding embodiments, wherein the at least one ingredient is added after the fermentation step.

P39. The product according to any of the three preceding embodiments, wherein the at least one ingredient is added after: the fermentation step and a heat treatment is performed

P40. The product according to any of the preceding embodiments, wherein the product comprises at least one food additive comprises at least one of:
preservative,
flavor enhancer,
colorant,
emulsifier,
stabilizer,
thickener, and
sweetener.

P41. The product according to any of embodiments P1 to P39, wherein the product is free from additives, particularly free of texturizing elements such as forms of:
microcrystalline cellulose (E460),
methyl cellulose (E461),
ethyl cellulose (E462),
hydroxypropyl cellulose (E463),
hydroxylpropyl methyl cellulose (E464),
ethyl methyl cellulose (E465),
sodium carboxy methyl cellulose (E466),
cross-linked carboxy methyl cellulose (E468),
enzymatically hydrolysed arboxy methyl cellulose (E469) and
hydroxyethyl cellulose (E1525).

Below, method embodiments will be discussed. These embodiments are abbreviated by the letter "M" followed by a number. When reference is herein made to a method embodiment, those embodiments are meant.

M1. A method for producing a mycelium-containing product, the method comprising inoculating a fermentable substrate with at least one fungal strain, and
fermenting the inoculated the fermentable substrate to generate a composite product comprising a mycelium-containing biomass.

M2. The method according to any of the preceding method embodiments, wherein the mycelium-containing product comprises a fungal mycelium and a fungal mycelium-containing biomass on the fermentable substrate, wherein the mycelium-containing biomass comprises a non-toxic edible fungal mycelium.

M3. The method according to the preceding embodiment, wherein the method comprises performing the fermenting step providing to the fermentable substrate the at least one fungal strain comprising at least one filamentous fungus from Ascomycota and/or Zygomycota phylum, preferably from a genus selected from t a group comprising Aspergillus, Rhizopus, Fusarium, Neurospora and Penicillium

M4. The method according to the preceding embodiment, wherein the at least one filamentous fungus comprises species selected from a group comprising Aspergillus oryzae, Rhizopus oryzae, Rhizopus oligosporus, Fusarium venetatum, Penicillium camemberti, Neurospora Intermedia, and Neurospora crassa.

M5. The method according to any of the two preceding embodiments, wherein the at least one filamentous fungus comprises a fungus selected from at least one of groups of
Halophiles such as Talaromyces leycettanu,
Thermophiles such as Phycomycetaces blakesleeanus, and
Acidophiles such as Aureobasidium pullans.

M6. The method according to any of the preceding method embodiments, wherein the fungal mycelium-containing biomass comprises at least one fungal strain from Basidiomycetes division, preferably from a genus selected from a group comprising Agaricus, Trametes, Fomes, Ganoderman, Pleurotus, Pholiota, Lentinula, Ganoderma, Hericium, Morchella.

M7. The method according to the preceding embodiment, wherein the at least one fungal strain comprises a species of: Agaricus bisporus, Trametes versicolor, Fomes fomentarius, Ganoderma sessile, Pleurotus pulmonarius, Pleurotus ostreatus, Pholiota nameko, Lentinula edodes, Ganoderma lucidum, Ganoderma sinense, Hericium coralloides, Hericium erinaceus, Pleurotus eryngii, and Morchella esculenta.

M8. The method according to any of the preceding method embodiments, wherein the method comprises growing fungal mycelium, wherein the method further comprises providing to the fermenting step at least one at least one nutrient suitable for growing the fungal mycelium.

M9. The method according to the preceding embodiment, wherein the at least one nutrient is comprised by the fermentable substrate.

M10. The method according to any of the preceding method embodiments, wherein the fermentable substrate comprises a substrate type comprising at least one of
Secondary output of food industry,
Organic substrates from legume family,
Pulses from legume family, and
Pulse derivatives.

M11. The method according to the preceding embodiment, wherein the pulses comprise at least one of:
soy,
white beans,
chickpeas,
red beans,
broad beans,
quinoa, and
peas.

M12. The method according to embodiment M10, wherein the secondary output of food industry comprises at least one of
okara,
beetroot pomace,
apple pomace,
tomato pomace,
textured vegetable proteins,
wheat bran,
coconut press cake,
rapeseed oil press cake,
linseed oil press cake, and
chickpea bran.

M13. The method according to any of the preceding method embodiments, wherein the method comprises controlling at least one fermentation parameter comprising at least one of:
temperature between 25 °C and 35 °C,
relative humidity between 35% and 95%,

M14. The method according to any of the preceding method embodiments, wherein the fermentable substrate comprises a substrate geometry comprising a particle geometry comprising particle sizes between 0.0.01 mm and 50 mm, preferably between 0.05 mm and 30 mm, more preferably between 0,05 mm and 10 mm.

M15. The method according to the preceding embodiment and with the feature of embodiment M10, wherein the fermentable substrate geometry is different from an original shape of the fermentable substrate type.

M16. The method according to any of the preceding method embodiments and with the features of the preceding embodiment, wherein the method comprises changing the original shape of the fermentable substrate type into the fermentable substrate geometry comprising an arrangement as particles comprising at least one of
flake-like three-dimensional geometry particles;
rod-like particles elongated in a first dimension and thin along at least one of: a second dimension and a third dimension;
thread-like particles elongated in a first dimension and thin along at least one of: a second dimension and a third dimension;

M17. The method according to any of the preceding method embodiments, wherein the method comprises providing the product with a plurality of air pockets comprising an air pocket diameter between 0.05 mm and 5 mm.

M18. The method according to any of the preceding method embodiments and with features of the preceding embodiment, wherein the method comprises controlling the fermentable substrate geometry.

M19. The method according to any of the preceding method embodiments, wherein the method comprises adding at least one functional ingredient with a concentration of at least 0.005% w/w, preferably between 0.01% w/w and 3% w/w.

M20. The method according to the preceding embodiment, wherein the at least one functional ingredient comprises at least one of:
starch such as modified corn starch,
fiber such as bam,
gum such as acacia gum and konjac gum, and
hydrocolloids such as alginate.

M21. The method according to any of the two preceding method embodiments, wherein the at least one functional ingredient comprises at least one of:
Bacterial polysaccharides such as Gellan, Xanthan;
Carrageenan such as Iota-carrageenan, Kappa-carrageenan;
Galactomannan such as Locust bean gum, Guar gum; and
Fibers such as Psyllium, Bamboo, Oat.

M22. The method according to any of the two preceding embodiments, wherein the method comprises incorporating the at least one functional ingredient into the fermentable substrate prior to the fermenting step.

M23. The method according to any of embodiments M19, M20 or M21 and with features of the previous embodiment, wherein the method comprises incorporating the at least one functional ingredient into the fermentable substrate after the fermenting step.

M24. The method according to the preceding embodiment, wherein the method comprises adding the at least one ingredient before the fermenting step.

M25. The method according to any of the two preceding embodiments, wherein the method comprises adding the at least one ingredient after the fermenting step and after a treatment step.

M26. The method according to any of the preceding method embodiments, wherein the method comprises adding at least fatty oil in ranges between 0.1% w/w and 3% w/w.

M27. The method according to the preceding embodiment, wherein the at least one fatty oil comprises at least one of:
deodorized coconut oil,
linseed oil,
rapeseed oil,
pumpkin seed oil,
microalgae derived oil such as oil from microalgae Schizochytrium sp., and macroalgae derived oil

M28. The method according to any of the two preceding embodiments, wherein the method comprises producing fish flavors by burning or heating up the at least one fatty oil.

M29. The method according to the preceding embodiment, wherein the heating step comprises heating between 50 °C and 220 °C for at least 1 minutes, preferably between 5 and 15 min.

M30. The method according to any of the preceding embodiments, wherein the method comprises adding at least one of: eicosapentaenoic acid (EPA), and docosahexaenoic acid (DHA) in a concentration of at least 1 microgram/g, preferably above 5 micrograms/g.

M31. The method according to any of the preceding method embodiments, wherein the method comprises adding at least one supplementary compound comprising at least one of: vitamin, and mineral.

M32. The method according to the preceding embodiment, wherein the at least one vitamin comprises at least one of:
vitamin B such as B-1, B-2, B-3, B-5, B-6, B-7, B-9, and B-12;
vitamin C;
vitamin A;
vitamin D;
vitamin E; and
vitamin K.

M33. The method according to any of the two preceding embodiments, wherein the at least one mineral comprises at least one of: sodium selenite, potassium iodide, calcium, potassium, sodium, chloride, phosphorus, magnesium, iron, copper, fluoride, selenium, zinc, chromium, molybdenum, iodine, and manganese.

M34. The method according to any of the preceding method embodiments, wherein the method comprises producing the fermentable substrate for the fermenting step, the method comprising at least one of:
grinding at least one ingredient to obtain a grinded material;
milling at least one ingredient to obtain a milled material;
reshaping at least one of: the grinded material and the milled material; and
blending at least one of: the grinded material and the milled material.

M35. The method according to the two preceding embodiments, wherein the method comprises controlling the fermentable substrate geometry by reshaping at least one of: the grinded material and the milled material.

M36. The method according to the three preceding embodiments, wherein the controlling step comprises the blending step preceding the reshaping step.

M37. The method according to any of the preceding method embodiments, wherein the method comprises controlling a particle size of the at least one ingredient.

M38. The method according to any of the preceding method embodiments and with the features of embodiment M18, wherein the method comprises controlling a product porosity via controlling the plurality of air pockets.

M39. The method according to any of the preceding method embodiments, wherein the method comprises for the fermenting step providing to the fermentable substrate a culture supplement comprising at least one nutrient comprising at least one of
peptones,
sodium aspartate,
malt extract,
nutritional yeast, and
ammonium nitrate

M40. The method according to any of the preceding method embodiments, wherein the method comprises adjusting a product flavor by adding at least one flavoring component comprising at least one of: vegan compound, artificial compound, algae derived compound, and natural industrially available compound.

M41. The method according to any of the preceding method embodiments and with the features of embodiment M9, wherein the fungal mycelium growing comprises a controlled growing.

M42. The method according to any of the preceding method embodiments, wherein the fermenting step is a solid-state fermentation process.

M43. The method according to any of the preceding method embodiments, wherein the fungal mycelium-containing biomass is at least partially hydrophobic.

M44. The method according to any of the preceding method embodiments, wherein the fermenting step comprises a fermentation temperature above 25 °C, preferably 25 °C and 35 °C, more preferably between 27 °C and 31 °C.

M45. The method according to any of the preceding method embodiments, wherein the method comprises controlling a product shape comprising one of: a square shape, a fish shape, seafood, fish, eel, prawn, shrimp, Cod fillet, Salmon fillet, Bream fillet, chicken breast, chicken leg, pork fillet, square, meat fillet, meat strip, fish piece, fish burger, shrimp, prawn, Saint Jacques, Scallop, fish, grass carp fillet, Peruvian anchoveta fillet, Silver carp fillet, Common carp fillet, Alaska pollock fillet, Nile tilapia fillet, Bighead carp fillet, Tuna fillet, and seabass fillet.

M46. The method according to any of the preceding method embodiments, wherein the method comprises providing the mycelium-containing product with a product texture resembling at least one of: seafood texture, fish texture, squid texture, eel texture, prawn texture, shrimp texture, Saint Jacques scallop texture, Cod fish texture, Salmon texture, Atlantic pollock texture, Bream texture, chicken breast texture, chicken leg texture, Beef fillet texture, pork fillet texture, seafood, fish, eel, prawn, shrimp, Cod fillet, Salmon fillet, Bream fillet, chicken breast, chicken leg, pork fillet, square, meat fillet, meat strip, fish piece, fish burger, shrimp, prawn, Saint Jacques, Scallop, fish, Grass carp fillet, Peruvian anchoveta fillet, Silver carp fillet, common carp fillet, Alaska pollock fillet, Nile tilapia fillet, Bighead carp fillet, Tuna fillet, Seabass fillet.

M47. The method according to any of the preceding method embodiments and with the features of embodiment M42, wherein the method comprises controlling at least one process parameter of the solid-state fermentation process, the at least one process parameter comprising at least one of:
water content of the fermentable substrate,
airflow,
humidity of the fermentable substrate,
pH of the fermentable substrate,
carbon dioxide content, and
fermentation temperature

M48. The method according to any of the preceding method embodiments, wherein the method comprises reducing and/or eliminating microbial contamination in the fermentable substrate by means of at least one substrate pre-treatment comprising at least one of:
sterilization,
pasteurization, and
pH adjustment in the fermentable substrate.

M49. The method according to the preceding embodiment, wherein the controlled growing comprises growing the mycelium substantially linear.

M50. The method according to any of the preceding method embodiments and with features of embodiment M41, wherein the controlled growing comprises a rhizomorphic growing.

M51. The method according to any of the preceding method embodiments, wherein the mycelium-containing product is according to any of the preceding product embodiments.

M52. The method according to any of the preceding method embodiments, wherein the method comprises casting the product after the fermenting step.

M53. The method according to any of the preceding method embodiments, wherein the method comprises fermenting the fermentable material on a substrate in a physical cast or mold.

M54. The method according to the preceding embodiment, wherein the physical cast or mold comprises a plurality of holes.

M55. The method according to any of the two preceding embodiments, wherein the method the physical cast or mold comprising a surface texture mimicking a surface texture of an animal product.

M56. The method according to any of the preceding method embodiments, wherein the method comprises
processing the product by at least one of: grinding, slicing or extruding the composite product into pieces of a custom geometry, and
binding the pieces together, wherein the binding comprises at least one of: an ordered binding manner, or disordered binding manner.

M57. The method according to preceding embodiment, wherein the method comprises binding the pieces by incorporating a binding liquid mixture comprising at least one binding agent.

M58. The method according to the preceding embodiment, wherein the at least one binding agent comprises at least one of: transglutaminases, hydrocolloids, sodium alginate, carrageenan, modified starches, methylcellulose or vegetable protein powders.

M59. The method according to any of the two preceding embodiments and with the feature of embodiment M2, wherein the method comprises
processing the composite after the fermenting step by slicing the composite into pieces of a custom geometry, and
binding the pieces by allowing the fungal mycelium to regrow in between the pieces in a second fermentation step to produce the product.

M60. The method according to any of the preceding method embodiments, wherein the method comprises processing the composite after the fermenting step by at least one of: cutting, grinding, slicing, or extruding to obtain fibrils with an axial dimension of at least 1 mm, preferably 3 mm, most preferably above 5 mm, and a diameter below 8mm, preferably 5 mm, most preferably 1 mm.

M61. The method according to any of the preceding method embodiments, wherein the method comprises joining together mycelium composite fibrils comprised by the in an aligned manner, wherein the mycelium composite fibrils comprise a longitudinal direction, wherein the method comprises aligning the longitudinal direction by
using at least one binder, or
forming an anisotropic matrix by regrowing the fungal mycelium in a second fermentation to produce the product.

M62. The method according to the preceding embodiment, wherein the method comprises distributing in between the joined fibrils at least one fat-containing ingredient.

M63.The method according to any of the preceding method embodiments, wherein the method comprises fermenting the fermentable material in a mold comprising a custom surface texture, including an overall 3D shape.

M64. The method according to the preceding embodiment, wherein the mold is produced by using at least one of: silicon, thermoforming, and 3D printing.

M65. The method according to any of the preceding method embodiments, wherein the method comprises fermenting the fermentable material in a mold comprising a negative of an animal origin protein product.

M66. The method according to the preceding embodiment, wherein the negative of the animal protein origin product comprises enhanced surface with respect to surface features of the animal origin protein product.

M67. The method according to any of the preceding method embodiments, wherein fermentation of the inoculated substrate in a mold that creates incisions/indentations in the product of at least 0.1 mm, preferably between 1 mm and 10 mm.

M68. The method according to any of the preceding method embodiments, wherein the method comprises at least one of: agitating or mixing the inoculated substrate, coating the mycelium after a first fermenting step to generate coated particles, and placing the coated particles in a mold.

M69. The method according to the preceding embodiment, wherein the method comprises coating the mycelium with at least one functional ingredient.

M70. The method according to any of the preceding method embodiments, wherein the method comprises providing at least one food additive.

M71. The method according to the preceding embodiment, wherein the at least one food additive comprises at least one of:
preservative,
flavor enhancer,
colorant,
emulsifier,
stabilizer,
thickener, and
sweetener.

M72. The method according to any of the two preceding embodiments, wherein the method comprises adding the at least one food additive during the fermenting step.

M73. The method according to any of embodiments M1 to M69, wherein the method comprises producing the mycelium-containing product free from additives, particularly free of texturizing elements such as forms of:
microcrystalline cellulose (E460),
methyl cellulose (E461),
ethyl cellulose (E462),
hydroxypropyl cellulose (E463),
hydroxylpropyl methyl cellulose (E464),
ethyl methyl cellulose (E465),
sodium carboxy methyl cellulose (E466),
cross-linked carboxy methyl cellulose (E468),
enzymatically hydrolysed arboxy methyl cellulose (E469) and
hydroxyethyl cellulose (E1525).

M74. The method according to any of the preceding method embodiments, wherein in the fermenting step, the method comprises covering the inoculated substrate with at least one removable covering component.

M75. The method according to the preceding embodiment, wherein the at least one removable covering component comprises a membrane comprising a plurality of porous to allow for oxygen, heat and humidity exchange.

M76. The method according the preceding embodiment, wherein the membrane comprises a layer of a polymeric film.

M77. The method according to any of the preceding embodiments, wherein the method comprises mixing at least one particle comprising the particle sizes, preferably at least two particles comprising the particle sizes.

M78. The product according to the preceding embodiment, wherein the at least two particles comprise different particle sizes.

Below, system embodiments will be discussed. These embodiments are abbreviated by the letter "S" followed by a number. When reference is herein made to a system embodiment, those embodiments are meant.

S1. A system for producing a mycelium-containing edible composite product, wherein is configured to ferment a fermentable substrate to generate a composite product comprising a mycelium-containing biomass, wherein the fermentable substrate is inoculated with at least one fungal strain.

S2. The system according to the preceding embodiment, wherein the mycelium-containing edible composite product is according to any of the preceding product embodiments.

S3. The system according to any of the preceding system embodiments, wherein the system comprises a tray bioreactor configured to ferment a substrate inoculated with at least one fungal strain capable of producing a non-toxic edible fungal mycelium.

S4. The system according to any of the preceding system embodiments, wherein the system comprises a solid-state drum bioreactor configured to provide movement to particles during mixing step.

S5. The system according to any of the preceding system embodiments, wherein the system comprises a continuous or semi-continuous solid-state fermentation bioreactor.

S6. The system according to any of the preceding system embodiments, wherein the system comprises at least one fermentation tray comprising a 3D shape.

S7. The system according to the preceding embodiment, wherein the at least one fermentation tray comprises at least one hole configured to allow aeration.

S8. The system according to any of the two preceding claims, wherein the at least one fermentation tray is shaped in form of an animal-origin product.

S9. The system according to the preceding embodiment, wherein the form comprises at least one of: seafood, fish, squid, eel, prawn, shrimp, Saint Jacques scallop, Cod, Salmon texture, Atlantic pollock, Bream, chicken breast, chicken leg, Beef fillet, pork fillet, Cod fillet, Salmon fillet, Bream fillet, chicken breast, chicken leg, pork fillet, square, meat fillet, meat strip, fish piece, fish burger, shrimp, prawn, Saint Jacques, Scallop, fish, Grass carp fillet, Peruvian anchoveta fillet, Silver carp fillet, common carp fillet, Alaska pollock fillet, Nile tilapia fillet, Bighead carp fillet, Tuna fillet, Seabass fillet.

S10. The system according to any of the preceding system embodiments and with features of embodiment S3, wherein comprises at least one removable covering component configured to cover the inoculated substrate.

S11. The system according to the preceding embodiment, wherein the at least one removable covering component comprises a membrane comprising a plurality of porous to allow for oxygen, heat and humidity exchange.

S12. The system according the preceding embodiment, wherein the membrane comprises a layer of a polymeric film.

S13. The system according to any of the preceding system embodiments, wherein the system is configured to carry out the method steps according to any of the preceding method embodiments.

M79. The method according to any of the preceding method embodiments, wherein the method comprising prompting the system according to any of the preceding system embodiments to perform any of the steps of the method according to the any of the preceding embodiment to produce the product according to any of the preceding product embodiments.

U1. Use of the system according to any of the preceding system embodiments for carrying out the method according to any of the preceding method embodiments.

The present invention will now be described with reference to the accompanying drawings which illustrate embodiments of the invention. These embodiments should only exemplify, but not limit, the present invention.
- Figs. 1A-C: schematically depict a mycelium-containing edible product comprising a shape according to embodiments of the present invention;
- Fig. 2: schematically depicts a processing of a substrate according to embodiments of the present invention;
- Figs. 3A-B: schematically depict composites comprising different substrates particles according to embodiments of the present invention;
- Figs. 4A-C: schematically depict addition of functional ingredient to the composite according to embodiments of the present invention;
- Figs. 5A-D: schematically depict steps for producing the product according to embodiments of the present invention;
- Figs. 6A-B: schematically depict placing and fermenting of the fermentable substrate into a mold or cast according to embodiments of the present invention;
- Figs. 7A-C: depict photographs of the product according to embodiments of the present invention with a plurality of shapes mimicking shapes of a plurality of animal origin protein products.

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

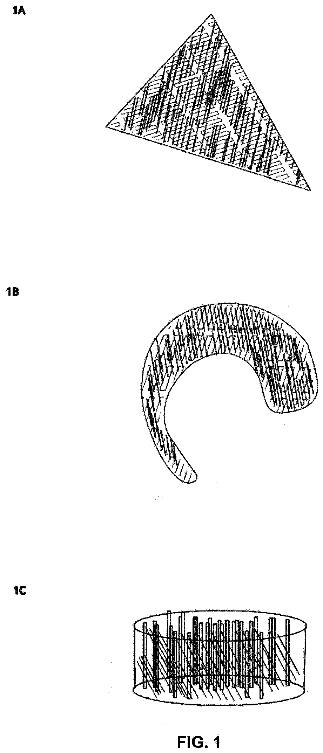

Figs. 1A-C conceptually depicts a white fish filet, a shrimp, and a filet. In simple terms, Figs. 1A-C depict a zoom on particles comprised by the product according to embodiments, of the present invention, wherein the particles comprise a given shape, which can be obtained according to the steps of the method of the present invention.

It should be understood that in Figs. 1A-C the inclined rectangles are intended to represent substrate particles and the vertical lines are intended to represent the mycelium. It should be understood that the vertical lines are not intended that to depict that the mycelium will have the depicted orientation.

Figs. 1A-C depict a conceptual representation of mycelium-containing edible products, wherein the product comprises a fungal mycelium-based biomass on a substrate. In one embodiment, the product may comprise a substrate in form of particulate matter such as round circles, exemplified with a rectangular shape in Fig. 1A, which may be inoculated and placed on a mold or cast. For instance, the mold or cast may comprise a shape that is intended to be provide to a final product. The fungus mycelium may then grow in and into the fermentable substrate particles binding the particles together to produce a single piece.

Figs. 1A-C depict a plurality of different applications of the product such as a fish filet whole cut, a shrimp and a filet mignon type steak. In particular, Fig. 1A depicts an example of the product according to embodiments of the present invention, in which a particulate substrate with a specific particle geometry, for instance as depicted in Fig. 1A, which comprises particles in a shape according to embodiments of the present invention inoculated, wherein the fungal mycelium may grow and be shaped by using the mold/cast, which would allow to obtain a fish filet. In another embodiment, the product may also be provided with a shape of shrimp such a depict in Fig. 1B, or a steak as shown in Fig. 1C, in which case the biomass or the fermentable substrate may be shaped into thin elongated fibrils comprising characteristics according to embodiments of the present invention, which is particularly beneficial as it results in a final product comprising a filet mignon type like appearance. More particularly, Fig. 1C depicts as substrate that was inoculated and then a block of substrate/mycelium composite was grown, followed by a cutting into stripes and put the stripes together, and fermented for an extra period of time to bind them together.

It should be understood that the product according to embodiments of the present invention comprises a given shape mimicking an animal origin product and thus, it also comprises other similar characteristics such as flavor and texture, which are provided to the product according to embodiments of the present invention. It should also be understood that in one embodiment, the product according to embodiments for the present invention may also be a product on its own, i.e., with characterizing feature of the product itself and not necessarily those mimicking features of animal origin products.

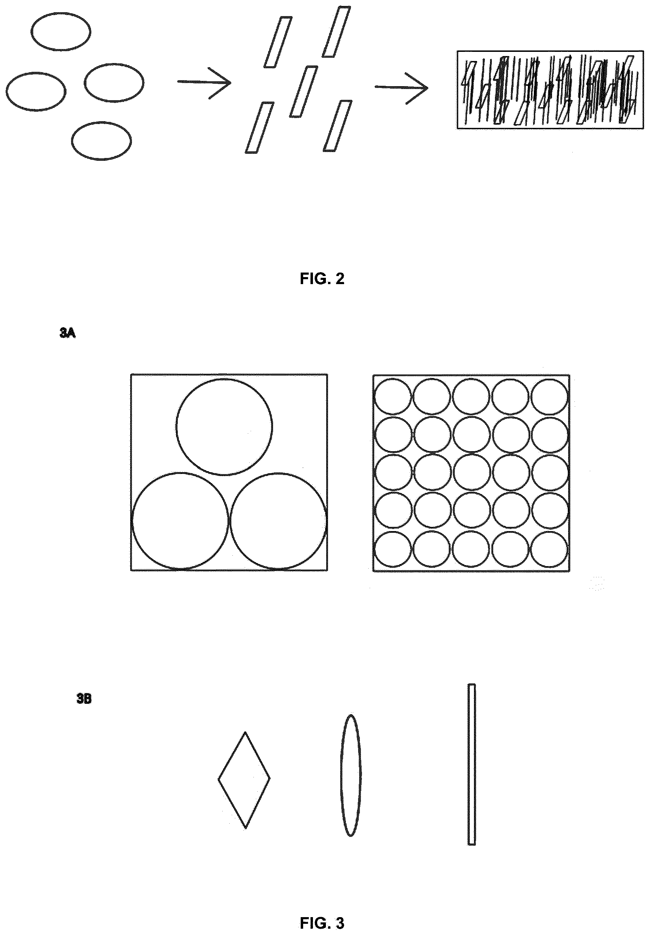

Fig. 2 depicts a process in which a starting substrate material such as white beans is, for instance, processed by grinding, milling or otherwise, to modify the geometry of the particle to obtain smaller substrate particles and/or substrate particles comprising a specific shape, such as long elongated particles. The processed substrate may then be inoculated with at least one fungal strain and placed in a container where the particles are still loose. The filled container may then be placed in a device for fermentation such as a fermenter at a fermentation temperature such as above 25 °C, preferably 25 °C and 35 °C, more preferably between 27 °C and 31 °C and humidity between 35 and 9t5% for several hours, time during which the growth of the mycelium may take place and lead to a single piece which is a composite matrix of the fungal mycelium and the processed substrate particles.

Fig. 3 conceptually depicts two different composites according to embodiments of the present invention, wherein the size of the fermentable substrate particles may be different between the two composites. In particular, Fig. 3 discloses different sizes or shapes that may lead to plurality of compactness levels, which is notably advantageous as it allows obtaining different amounts of void space. Fig. 3A depicts two configurations in which the particle size is different for each configuration. In simple terms, Fig. 3a depicts spherical particles, wherein the larger the particle, the more void space is left in between the particles with respect to a more compact configuration with smaller particles. Fig. 3B depicts different particle geometries according to embodiments of the present invention, such as a rhomboidal, long fibril, random particle. Rhomboidal particles are particularly advantageous, as these allows to obtain a flaky product, and long fibrils are particularly advantageous as these allows to obtain a product resembling directional muscle tissues of product of animal origin such as cow or chicken meat.

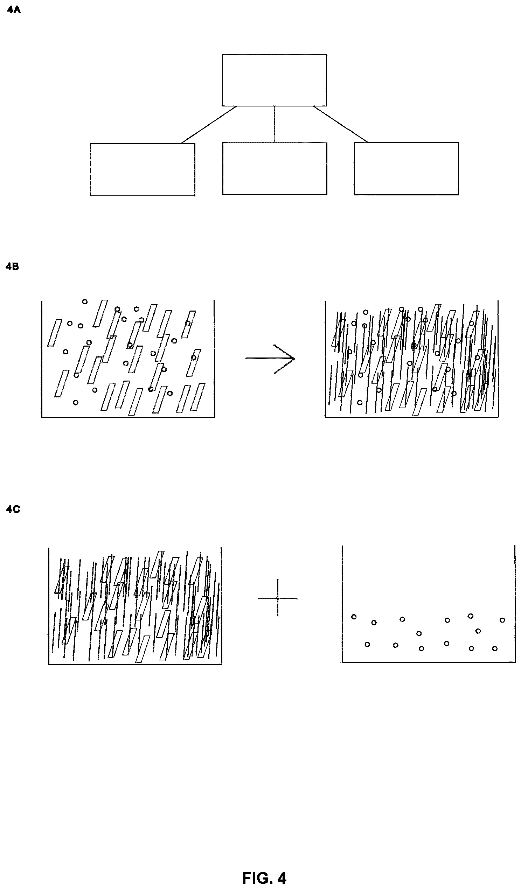

Fig. 4A conceptually depicts a diagram, wherein addition of functional ingredients according to embodiments of the present invention. For instance, functional ingredients may be added to the edible mycelium composite as powders, which is notably advantageous as it may affect different properties of the composite such as mechanical; organoleptic properties such as mouthfeel, flavor and aroma; as well as food functionality attributes such as water retention, oil absorption, water release upon cooking, among others. The functional ingredients may include starches, modified starches, protein isolates, functional proteins, fibers, among other. Additional or alternative, the functional ingredients may also comprise a material that may serve as supplement for the fungal growth, which particular beneficials as may positively affect a plurality of factors such as growth speed, primary and secondary metabolites, and hyphal properties, which further allows to influence the aforementioned mechanical, organoleptic and food functional properties. The modification of the composite can be implemented by incorporating further additional functional ingredients prior to or after fermentation. For example, Iota Carrageenan can be added prior to fermentation, which is notably advantageous as it allows to obtain a slithery mouthfeel in the end product. Similarly, bamboo fibers may be added, which is particularly beneficial to increase water retention and fibrosity sensation. Culture supplements such as nutritional yeast may also be added, which is notably advantageous to increase nitrogen source, which is further particularly beneficial, as it leads to an increase protein content of the final product.

Fig. 4B conceptually depicts an implementation of a method according to embodiments of the present invention, wherein addition of powdered functional ingredients and/or culture supplements may be performed prior to fermentation. A mix of the inoculated substrate particles together with the powdered functional ingredients may then be placed into the appropriate container and fermented. Fig 4C. conceptually depicts a method according to embodiments of the present invention, wherein functional ingredients may be introduced after the fermentation of edible mycelium composite, where such ingredients are added into a liquid phase, which is then incorporated into the product, which may, for instance, comprise using vacuum impregnation.

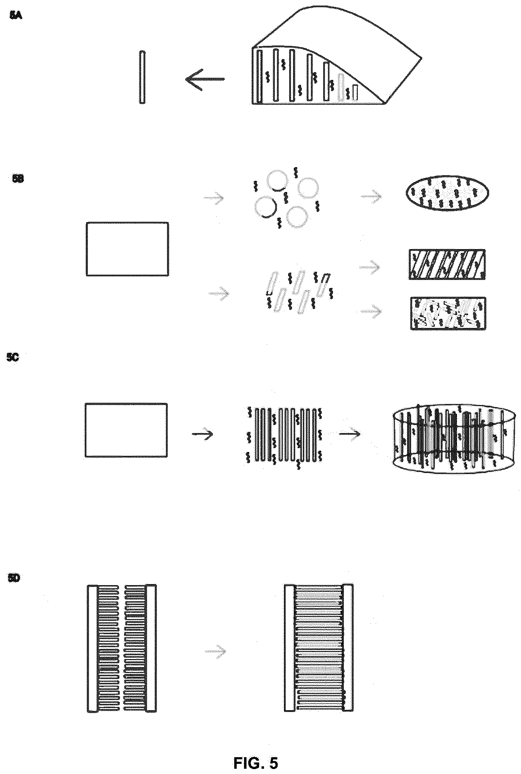

Fig. 5 schematically depicts a process of making the product comprising the edible mycelium composite according to embodiments of the present invention, wherein after a first fermentation, the edible mycelium composite may, for example, cut, broken, separated and/or extruded into pieces of a determined geometry. In one embodiment, these pieces may then be bound together by using a binding agent such as a binding liquid, or by regrowing the mycelium in a second fermentation, wherein the second fermentation is shorter than the first fermentation.

Fig. 5A conceptually depicts a serving piece of a fish filet comprises a plurality of pieces which were made by cutting or slicing the edible mycelium composite, which may be then arranged in, for example, a form of flakes bound together to conform to a fish filet. In one embodiment, the flake-like pieces of the edible mycelium composite may be bound to each other by introducing in between the pieces a liquid containing a binding agent, wherein the liquid may comprise a combination of at least one binding agent comprising, for example, at least one of: hydrocolloids, methylcellulose, potato proteins, starches and oils. Fig. 5B conceptually depicts an example in which the edible mycelium composite is processed after fermentation into long and thin in diameter strands, similar to muscle fibers of animal tissue. Such composite particle strands may then be placed together in a mold, or any container that keeps the pieces in close contact and the binding liquid coats each particle. Fig. 5C schematically depicts a process in which an already fermented edible mycelium composite block may be cut, sliced and/or separated into pieces and the pieces may be bound together into a block by using a binding agent such as a binding liquid. This approach is particularly advantageous, as it results in a block that is no longer uniform but rather composed of multiple weakly bound pieces that may separate into such pieces upon applying mechanical forces. Fig. 5D schematically depict a method for binding the pieces through a second short fermentation. In one embodiment, during this second fermentation, a new mycelium may be created emanating from each individual piece and reaching the neighboring pieces, which is notably advantageous, as it allows binding the pieces together without utilizing any additives and thus provides a method to replace additives and binding agents. In particular, Fig. 5D depicts an example in which the edible mycelium composite is processed after the first and main fermentation into long and thin in diameter strands, similar to muscle fibers of animal tissue. Such composite particle strands may then be placed together in a mold, or any container that keeps the pieces in close contact. The mycelium growing from each side will eventually reach and grow on the opposite side binding the pieces together.

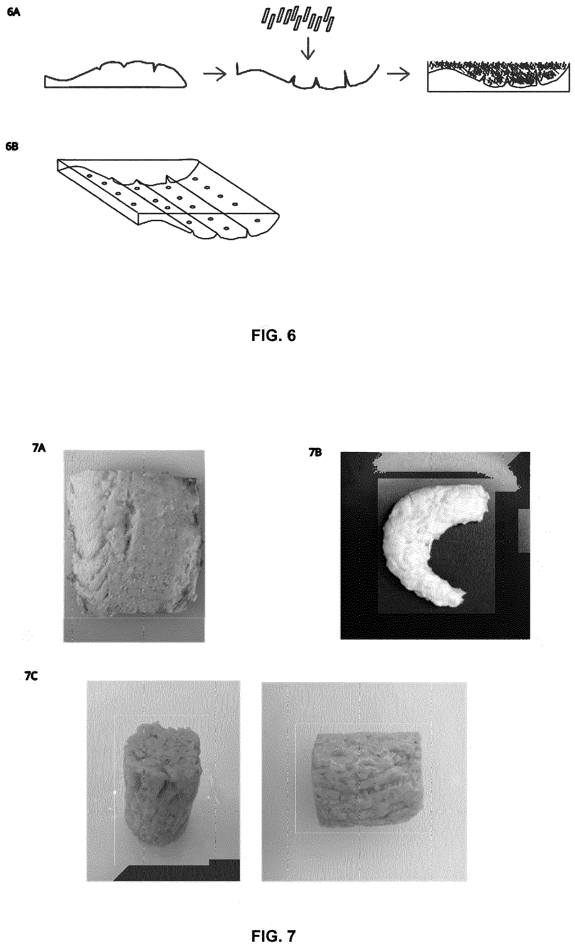

Fig. 6 schematically depicts a process in which the inoculated substrate particles are placed into a mold or cast and fermented in the mold or cast. In one embodiment, the cast may be used to impart a shape, a surface texture and geometrical features such as indentations. The surface texture is advantageous to produce a mouthfeel that is different to that of a rectangular block or uniform surface, and depending on that surface texture the feeling in the mouth upon consumption, for example by contact with the tongue may be reminiscent of one or other animal product. For example, in the case of fish, the cast may provide a surface feature similar to that occurring in the myosepta, which give a mouthfeel reminiscent of fish. Furthermore, if such surface details are made to be deep indentations, application of mechanical forces such as cutting, may lead to a preferential breakage of the product in those regions, which is notably advantageous as this provides a mechanical property reminiscent of fish. Fig. 6A depicts a schematic cross section of a fish filet (left), the inverted cross section (middle) and the arrangement in which a mold, following the contour of the fish surface is placed into a container, and the fungus inoculated substrate particles are placed on top of the mold (right). The mold may be adapted to each strain. For example, Aspergillus oryzae needs stronger oxygenation and access to humidity than Rhizopus oryzae. Therefore, the permeability of the mold may be adapted, as well as perforations performed to give oxygen access. Such container may then be placed in an incubator to ferment and form the edible mycelium composite with the desired surface texture properties. In one embodiment, surface features may purposely be exaggerated in the mold, so that after pasteurization of the product, in which geometrical changes occur, the desired surface texture is still present. For example, indentations that form the axial lines of the fish are increased in depth in the mold comprising a depth of at least 0.1 mm, preferably between 1 mm and 10 mm. Fig. 6B depicts a 3D representation of the mold for the product mimicking a fish filet comprising a plurality of holes, which is notably advantageous as this allows for oxygenation during fermentation. Different fungal species may require different levels of oxygenation, and thus, the approach of the present invention is particularly beneficial, as it allows to provide a different size and density of the holes.

Figs. 7A-C depicts photographs of the product comprising a plurality of different shapes such as Fig. 7A mimics a white fish fillet produced by placing the inoculated substrate in a mold as shown in Fig. 6C, Fig. 7B mimics a shrimp produced in the same way but in a shrimp mold, and Fig. 7C mimics a filet mignon produced by cutting the edible mycelium composite intro stripes, placing the stripes together in an aligned manner, and fermenting for an extra hour to allow the mycelium to rebind the piece together similar to Fig. 5D. Moreover, the invention may also be described, but not limited to, in relation to following examples.

### Example 1: White fish fillet

Example 1 relates to a white fish fillet as conceptually depicted in Fig. 7A. In example 1, a white fish alternative is produced in the form of a white fish fillet, like that of a Hake fillet. White beans are chosen as substrate due to their white color. The dry white beans are ground using, for example, a malt grinding machine or a flour mill to obtain particles of sizes between 3 and 5 mm. The size range is obtained by sieving the ground dry bean particles through the appropriate sieves. This size is notably advantageous, as it provides to the final product a mouth feeling in which the different particles fall apart when eating, resembling the sensation of fish flakes falling apart. The white bean hulls particles are sorted out at this step since these provide an undesirable texture in the final product. The dehulled particles are soaked at 4°C for 12 hours, and then steamed for 45 min at 100°C and 100% humidity in a steaming oven. The particles are let cool to 37°C and then inoculated with Aspergillus Oryzae spores. The spore powder can be diluted in potato starch to improve growth. The inoculated substrate is placed on a silicon mold in the shape of a white fish fillet. The mold contains surface features of a filet, wherein the features have been enhanced in, for example, depth, by design. This particularly beneficial, as it allows producing both a specific mouthfeel upon contact with the tongue as well as a preferential breaking during cutting by the consumer. The mold contains perforations of approximately 1 mm in diameter spaced every 2.5 cm to allow for air exchange. The mold is 12 cm in the longitudinal dimension, 4 cm in the lateral dimension, and approximately 1.5 cm deep at the deepest point. The mold is placed in an incubator that has temperature and humidity control, for 28h at 80% humidity. After fermentation the mycelium composite is removed from the mold marinated with an emulsion containing a 0.5% rapeseed or linseed oil, an optimized mix of natural vegan aromas, which allows resembling white fish flavor, and an emulsifying agent such as xanthan gum at 1%. The marinade is applied in a proportion of, for example, 15% w/w of the weight of the edible composite, by placing both the solution and marinade in a plastic bag and vacuum sealing at 1 bar for 30 seconds. Through vacuum the marinade distributed homogeneously throughout the edible composite. The marinade it notably advantageous for providing a juicy mouthfeel as well as transporting the added aromas. The marinated mycelium composite is then pasteurized, for example, by using High Pressure Pasteurization or by placing the bag in a water bath at 95°C for 1h. This final product might be then used in multiple applications. These include, consuming the white fish fillet by pan-frying and serving with an accompaniment, or it might be coated in a batter, or any other applications. In one embodiment, the present invention also allows producing an increased gel-like slithery mouthfeel, such as that of the common Dab (Limanda limanda). For this purpose, a 1% w/w of iota carrageenan can be added. In another embodiment, it also possible to control the hardness of the product, for example, if fish of more hardness are desired, fermentation time can be increased to obtain a harder composite, for example by extending the fermentation time to 32h. In another embodiment, the same procedure is followed, with the exception that the substrate is composed of a 1:1 mix of two different particle sizes. For this, grinding of the dry white beans is performed. Resulting particles are sieved using stainless steel mesh sieves in two categories: particles of a size below 0.05 mm and particles of a size between 3 and 5 mm. The two particle sizes are mixed in a 1:1 ratio and the above procedure is followed in the exact same manner. The combination of two different particle sizes allows to create a final product with a specific texture, namely, that of a uniform phase that allows the material hold together therefore providing cohesiveness, while the larger particles provide a mouth feeling of pieces falling apart and therefore of a lighter and softer texture.

### Example 2: A shrimp

Example 2 relates to a shrimp as conceptually depicted in Fig 7B. Following the same procedure as described in example 1 with some modifications, a shrimp analogue can be produced. In this case, the fermentation time is extended to 36h to allow for the mycelium to further grow and consolidate in the substrate. In this way, the mycelium network becomes much tighter and therefore provides a harder bite and a chewier product, better resembling the organoleptic properties of a shrimp. In this case, the marinade vegan natural aromas are optimized for a shrimp-like taste. A coloring on the surface is applied to produce the characteristic visuals of shrimps.

### Example 3: Meat fillet

Example relates to a meat fillet. In example 3, a fibrous meat alternative in the form of a cylinder out of white medallions can be sliced. The fibrous meat alternative is produced by slicing the edible mycelium composite into small fibers and re-binding those fibers to form a fibrous meat analogue cylinder. The rebinding can be performed in two ways, by addition a binder (Fig 5C) or by a second fermentation process (Fig 5D). White beans, chickpeas, soy, or a combination of them may be chosen. The color of the substrate in this case is not relevant since the sample may afterwards be colored. The substrate is ground using, for example, a malt grinding machine or a flour mill to obtain particles of sizes between 1 and 2 mm. The size range is obtained by sieving the ground dry bean particles through the appropriate sieves. This size range is particularly beneficial to produce a compact matrix in which the mycelium and substrate particles are tightly interleaved. The bean hulls particles are sorted out at this step since they provide and undesirable texture in the final product. The dehulled particles are soaked at 4°C for 12 hours, and then steamed for 60 min at 100°C and 100% humidity in a steaming oven. The particles are let to cool to 37°C and then inoculated with Aspergillus Oryzae spores. The spore powder can be diluted in potato starch to improve growth. The inoculated substrate is placed on a rectangular tray of approximately 50 cm wide, 30 cm in depth and 5 cm thick. The substrate is placed onto the tray to form a layer 3 cm in thickness, leaving 2 cm free. The tray contains perforations of approximately 1 mm in diameter spaced every 2.5 cm to allow for air exchange and is covered on the top. The tray is placed in an incubator that has temperature and humidity control, for instance, for 32h at 80% humidity. After fermentation the mycelium composite is removed from the tray and sliced into fibers of 2cm in length and 1 mm in diameter. The stripes are aligned in their axial dimension to produce the fibrous meat analogue cylinder of 20 cm long and 7 cm in diameter, where the fibers are aligned to each other and with the axial dimension of the larger cylinder they form. The fibers can be coated prior to forming the meat analogue cylinder with a marinade containing a binder configured to join the fibers together. The binder containing marinade is, for example, a 1% w/w solution of sodium alginate along with natural vegan beef-like flavorings up to 2.5% w/w, food grade coloring including a combination of beetroot extract and carotin, and oil such as sunflower oil at 1% w/w. In one embodiment, at this point a fat analogue may be added or distributed between the fibers, by applying it to the fibers before forming the fibrous meat analogue cylinder. The fibrous meat analogue cylinder is subjected to heat treatment both for pasteurization as well as for setting the binders, for example, by vacuum packing the fibrous meat analogue and placing it in a water bath at 95°C for 1h or until the core temperature reaches 90°C. In another embodiment, the marinade coating and impregnating the mycelium composite fibers and containing the aromas and the binder can be applied not before, but after the fibers have been aligned to form the fibrous meat analogue cylinder. This is done by, for example, marinating the fibrous meat analogue cylinder in the marinade under vacuum and then proceeding with the heat treatment.

In another example, the same procedure is followed with the exception that the binding additives are avoided by carrying out a second fermentation after the mycelium composite block is sliced into the fibers. For this, the fermentation of the mycelium composite is first carried out for only 24 hours. Then cut into fibers as described in the previous paragraph and the meat analogue cylinder is formed the same way by aligning the fibers parallel to the cylinder axial dimension. No marinade is added at this point. The meat analogue cylinder is placed in the incubator to ferment in, for example, the same temperature and humidity conditions for an extra 4 hours. In this manner, the regrowth of the mycelium, emanating from each fiber, binds the fibers together and thus no binding agent is necessary.

The meat analogue cylinder therefore has two relevant binding strength parameters, the intra-fiber binding strength, that is, the binding strength of the fiber themselves, and the inter-fiber binding strength, that is, the binding strength among the fibers after being recombined together. While inter-fiber hardness and binding strength is determined by the total fermentation time, the inter-fiber binding strength is determined by the time of the second fermentation. In this way is it possible to adjust the relative binding strengths, for instance, as depicted in Fig. 7C.

After the second fermentation, a binding-agent free marinade is applied in a proportion of, for example, 15% w/w of the weight of the edible composite. The meat analogue of vacuum sealed together with the marinade. Through vacuum the marinade distributed homogeneously throughout the edible composite. The marinade is notably beneficial to provide a juicy mouthfeel as well as transporting the vegan natural flavoring. The fibrous meat analogue cylinder is subjected to heat treatment for pasteurization, for example, by vacuum packing the fibrous meat analogue and placing it in a water bath at 95°C for 1h or until the core temperature reaches 90°C.

### Example 4: Chicken-breast analogue

Example 4 relates to the edible mycelium composite made to produce chicken-breast analogue. In simple terms, de-hulled chickpeas are used as the substrate and prepared by soaking at 4°C for 12 hours and cooked in boiling water for 1h. The cooked chickpeas are inoculated with R. oryzae or A. oryzae spores in the form of a powder. The spore powder can be mixed 1: 1 w/w with potato starch, which is particularly beneficial for improving the growth of the mycelium. To the mix of spore powder and potato starch, peptones are added which promote higher density of the mycelium and also prevent sporulation. The inoculated substrate is then processed into a paste by means of a blending machine. The inoculated paste is spread in the form of a thing sheet in the corresponding solid state fermentation tray. The thin sheet is 50 cm wide, 30 cm deep, and between 1 mm in thickness. The tray is placed in an incubator machine capable of providing a controlled and stable temperature and humidity. The inoculated substrate is fermented at 30°C for 30h or until a uniform layer of mycelium is formed on top of the inoculated thin sheet. Addition of peptones is notably advantageous, as it allows achieving a thicker (at least 1 mm) and denser layer of mycelium forming on top of the thin sheet. After fermentation is completed, the fermented thin sheet is cut into strips of, for example, 15 mm in length and 3 mm wide. The stripes are mixed with a marinade including a binder configured to join the stripes together, yet not fully bound so that upon consumption and cutting with a knife, the material resembles a chicken breast. This binder containing marinade may, for example, be a 1% w/w solution of sodium alginate along with natural vegan chicken-like flavorings up to 2.5% w/w, xanthan gum at 1% and oil such as sunflower oil at 1% w/w. The coated/marinated stripes are placed in a cast, having the shape of, for example, a chicken breast. This cast containing the marinated stripes is cooked by steaming 95°C for 1h or until the core temperature reaches 90°C.

While in the above, a preferred embodiment has been described with reference to the accompanying drawings, the skilled person will understand that this embodiment was provided for illustrative purpose only and should by no means be construed to limit the scope of the present invention, which is defined by the claims.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. A mycelium-containing edible composite product, the product comprising a fungal mycelium-containing biomass grown on a fermentable substrate, wherein the mycelium-containing biomass comprises a non-toxic edible fungal mycelium.

2. The product according to the preceding claim, wherein the at least one fungal strain comprises at least one filamentous fungus from at least one of:
Ascomycota and/or Zygomycota phylum, preferably from a genus selected from a group comprising Aspergillus, Rhizopus, Fusarium, Neurospora and Penicillium, wherein the at least one filamentous fungus comprises species selected from a group comprising Aspergillus oryzae, Aspergillus sojae, Rhizopus oryzae, Rhizopus oligosporus, Fusarium venetatum, Penicillium camemberti, Neurospora Intermedia, and Neurospora crassa,
Basidiomycetes division, preferably from a genus selected from a group comprising Agaricus, Trametes, Fomes, Pleurotus, Pholiota, Lentinula, Ganoderma, Hericium, Morchella.

3. The product according to any of the preceding claims, wherein the fermentable substrate comprises at least one nutrient suitable for growing fungal mycelium, wherein the fermentable substrate comprises a substrate type comprising at least one of
secondary output of food industry,
organic substrates from legume family,
pulses from legume family, and
pulse derivatives.

4. The product according to any of the preceding claims, wherein the fermentable substrate comprises a substrate geometry comprising a particle geometry comprising particle sizes between 0.005 mm and 50 mm, preferably between 0.05 mm and 30 mm, more preferably between 0,05 mm and 10 mm, wherein the fermentable substrate comprises at least one particle comprising the particle sizes, preferably at least two particles comprising the particle sizes, wherein the fermentable substrate geometry is different from an original shape of the fermentable substrate type, wherein the particle geometry of the fermentable substrate geometry comprises at least one geometrical shape of: octahedral, cylindrical, spherical, pyramidal, tetrahedral, conical, rectangular prism, icosahedral, dodecahedral, cuboctahedral, helical, ellipsoid, parabolic, hyperbolic, rhombic dodecahedron, cone frustum, cuboctahedron, toroidal, triangular prism, elliptical cylinder, pentagonal prism, crescent, oblate spheroid.

5. The product according to any of the preceding claims, wherein the product comprises at least one of:
at least one flavoring component comprising at least one of: vegan compound, artificial compound, algae derived compound, and natural industrially available compound; and
at least one functional ingredient with a concentration of at least 0.005% w/w, preferably between 0.01% w/w and 3% w/w, wherein the at least one functional ingredient comprises at least one of:
starch such as modified corn starch,
fiber such as bamboo fibers,
gum such as acacia gum and konjac gum, and
hydrocolloids such as alginate.

6. A method for producing a mycelium-containing product, the method comprising
inoculating a fermentable substrate with at least one fungal strain, and
fermenting the inoculated the fermentable substrate to generate a composite product comprising a mycelium-containing biomass,
wherein the fermenting step is a solid-state fermentation process.

7. The method according to the preceding claim, wherein the mycelium-containing product comprises a fungal mycelium and a fungal mycelium-containing biomass on the fermentable substrate, wherein the mycelium-containing biomass comprises a non-toxic edible fungal mycelium, wherein the method comprises performing the fermenting step providing to the fermentable substrate the at least one fungal strain comprising at least one filamentous fungus from Ascomycota and/or Zygomycota phylum, preferably from a genus selected from group comprising Aspergillus, Rhizopus, Fusarium, Neurospora and Penicillium, wherein the at least one filamentous fungus comprises species selected from a group comprising Aspergillus oryzae, Rhizopus oryzae, Rhizopus oligosporus, Fusarium venetatum, Penicillium camemberti, Neurospora Intermedia, and Neurospora crassa.

8. The method according to any of the claims 6 to 7, wherein the method comprises growing fungal mycelium, wherein the method further comprises providing to the fermenting step at least one at least one nutrient suitable for growing the fungal mycelium, wherein the fermentable substrate comprises a substrate type comprising at least one of:
Secondary output of food industry,
Organic substrates from legume family,
Pulses from legume family, and
Pulse derivatives.

9. The method according to the preceding claim, wherein the method comprises changing an original shape of the fermentable substrate type into the fermentable substrate geometry comprising an arrangement as particles comprising at least one of:
flake-like three-dimensional geometry particles;
rod-like particles elongated in a first dimension and thin along at least one of: a second dimension and a third dimension; and
thread-like particles elongated in a first dimension and thin along at least one of: a second dimension and a third dimension.

10. The method according to any of claims 6 to 9, wherein the method comprises
adding at least one functional ingredient with a concentration of at least 0.005% w/w, preferably between 0.01% w/w and 3% w/w, wherein the at least one functional ingredient comprises at least one of: starch such as modified corn starch, fiber such as bam, gum such as acacia gum and konjac gum, and hydrocolloids such as alginate; and
adjusting a product flavor by adding at least one flavoring component comprising at least one of: vegan compound, artificial compound, algae derived compound, and natural industrially available compound.

11. The method according to any of claims 6 to 10, wherein the method comprises producing the fermentable substrate for the fermenting step, the method comprising at least one of:
grinding at least one ingredient to obtain a grinded material;
milling at least one ingredient to obtain a milled material;
reshaping at least one of: the grinded material and the milled material; and
blending at least one of: the grinded material and the milled material,
wherein the method comprises controlling the fermentable substrate geometry by reshaping at least one of: the grinded material and the milled material.

12. The method according to any of claims 6 to 11, wherein the method comprises at least one of:
processing the product by at least one of: grinding, slicing or extruding the composite product into pieces of a custom geometry, and binding the pieces together, wherein the binding comprises at least one of: an ordered binding manner, or disordered binding manner, wherein the method comprises binding the pieces by incorporating a binding liquid mixture comprising at least one binding agent, wherein the at least one binding agent comprises at least one of: transglutaminases, hydrocolloids, sodium alginate, carrageenan, modified starches or vegetable protein powders, wherein the method comprises;
processing the composite after the fermenting step by slicing the composite into pieces of a custom geometry, and binding the pieces by allowing the fungal mycelium to regrow in between the pieces in a second fermentation step to produce the product processing the composite after the fermenting step by at least one of: cutting, grinding, slicing, or extruding to obtain fibrils with an axial dimension of at least 1 mm, preferably 3 mm, most preferably above 5 mm, and a diameter below 8 mm, preferably 5 mm, most preferably 1 mm; and
joining together mycelium composite fibrils comprised by the in an aligned manner, wherein the mycelium composite fibrils comprise a longitudinal direction, wherein the method comprises aligning the longitudinal direction by using at least one binder, or forming an anisotropic matrix by regrowing the fungal mycelium in a second fermentation to produce the product, wherein the method comprises distributing in between the joined fibrils at least one fat-containing ingredient.

13. A system for producing a mycelium-containing product, wherein the system is configured to ferment a fermentable substrate to generate a composite product comprising a mycelium-containing biomass, wherein the fermentable substrate is inoculated with at least one fungal strain.

14. The system according to the preceding claim, wherein the system comprises
tray bioreactor configured to ferment the inoculated substrate with at least one fungal strain capable of producing a non-toxic edible fungal mycelium,
a solid-state drum bioreactor configured to provide movement to particles during a mixing step.

15. The system according to any of the preceding system claims, wherein the system comprises at least one fermentation tray comprising a 3D shape, wherein the at least one fermentation tray comprises at least one hole configured to allow aeration, wherein the at least one fermentation tray is shaped in form of an animal-origin product, wherein the form comprises at least one of: seafood, fish, squid, eel, prawn, shrimp, Saint Jacques scallop, Cod, Salmon texture, Atlantic pollock, Bream, chicken breast, chicken leg, Beef fillet, pork fillet, Cod fillet, Salmon fillet, Bream fillet, chicken breast, chicken leg, pork fillet, square, meat fillet, meat strip, fish piece, fish burger, shrimp, prawn, Saint Jacques, Scallop, fish, Grass carp fillet, Peruvian anchoveta fillet, Silver carp fillet, common carp fillet, Alaska pollock fillet, Nile tilapia fillet, Bighead carp fillet, Tuna fillet, Seabass fillet.
